## Europäisches Patentamt
## European Patent Office
## Office européen des brevets

(11) Publication number: **0 039 578**
**B1**

(12) **EUROPEAN PATENT SPECIFICATION**

(45) Date of publication of patent specification: **10.04.85**

(51) Int. Cl.⁴: **A 63 B 71/12**

(21) Application number: **81301901.5**

(22) Date of filing: **30.04.81**

(54) **Leg aid device.**

(30) Priority: **02.05.80 US 146142**

(43) Date of publication of application:
**11.11.81 Bulletin 81/45**

(45) Publication of the grant of the patent:
**10.04.85 Bulletin 85/15**

(84) Designated Contracting States:
**AT CH DE FR GB IT LI**

(56) References cited:
**DE-A-2 238 038**
**DE-C- 838 479**
**US-A-3 928 872**
**US-A-4 100 918**
**US-A-4 130 115**
**US-A-4 136 404**

(73) Proprietor: **Davis, Edward P.**
**1446 Delaware Avenue**
**St. Paul Minnesota 55118 (US)**

(72) Inventor: **Davis, Edward P.**
**1446 Delaware Avenue**
**St. Paul Minnesota 55118 (US)**

(74) Representative: **Booth, Philip Charles et al**
**LLOYD WISE, TREGEAR & CO. Norman House**
**105-109 Strand**
**London, WC2R OAE (GB)**

Courier Press, Leamington Spa, England.

## Description

This invention relates to a leg aid device for increasing the stamina of a leg repeatedly flexed and extended at the knee, and more particularly, to a leg aid device having biasing means and a combination of holders which effectively cause an arcuate contour for the biasing means during leg flexure.

The biasing means aids in supporting a portion of a user's body weight when the user's leg is flexed (i.e., bent). When the user extends his leg (i.e., straightens it), the biasing means springs back toward a straightened condition, and thereby aids the user's leg muscles in causing extension and resulting upward movement of the user's body. In effect, the leg muscles of the user are relieved from some, but not all, of the strain they undergo during flexure and extension.

The main usefulness of the device is to increase leg stamina without interfering with leg functions, without any significant leg torsion or twisting, as desired by skiers and others engaged in athletic or sport activity involving repeated leg flexure and extension.

Patent No. 838,479 of Bundesrepublik Deutschland teaches a knee articulation prosthesis of spring steel strips, presumably highly flexible, held by upper and lower holders which permit bending articulation of them at the knee area exclusively. Patent No. 3,928,872 of the United States of America teaches a leg aid of spring torsion bars, preferably permanently slightly bent, and presumably inherently stiff (but flexible to a limited degree), and held on lateral sides of a skier's leg by upper and lower holders exclusively. A hinged leg aid device for skiers is shown in Patent No. 4,136,404 of the United States of America. Some known orthopedic appliances cause torsion or twisting of a user's leg. No prior art leg appliance is known to have the properties and perform the functions of the devices taught herein.

According to the present invention, a leg aid device comprises elongated means for placement upon a user's leg and holding means for maintaining said elongated means upon the user's leg, said elongated means consisting essentially of elongated flexibly resilient biasing means having a length sufficient for placement in approximate alignment with the user's leg from a level near the ankle area up to at least the level of the mid-thigh area and having a flexibility sufficient for said length to be repeatedly yieldingly bent without taking on a permanent set, said biasing means when held upon a body-supporting user's leg by said holding means functioning to support a portion of the user's body weight during the times the user's leg is in a flexed condition and functioning to aid the user's leg muscles in causing extension of the user's leg from a flexed condition, said holding means consisting essentially of a lower holder for fastening the lower end of said biasing means at a level near the user's ankle area and an upper holder for maintaining the upper end of said biasing means at a level at least as high as the user's mid-thigh area, said device being particularly characterized in that said biasing means is capable of having its length repeatedly yieldingly bent into at least a semi-circle without taking on a permanent set while at the same time being sufficiently resistant to bending to provide support for an increasing portion of a user's body weight as the user's leg flexure increases, and by the further fact that said holding means consists also of an intermediate holder for applying a forward pulling force transversely upon said biasing means at a mid-portion thereof at the level of the user's knee area, said intermediate holder being separate from said lower and upper holders but functioning in cooperation therewith to cause said biasing means to urge the user's leg toward a straightened condition without significant torsion or twisting of the user's leg and also functioning in cooperation therewith during leg flexure to cause said biasing means to take on an arcuate contour throughout substantially the entire length of said biasing means.

The biasing means may comprise two members for placement at lateral portions of a leg. It may comprise a single member for placement behind a leg, which is advantageous for skiers riding chair lifts. Each biasing member preferably comprises a bundle or grouping of a plurality of elongated flexible elements.

Embodiments of the invention are shown in the accompanying drawings in which schematic illustrations are employed; and in all Figures, parts are broken away.

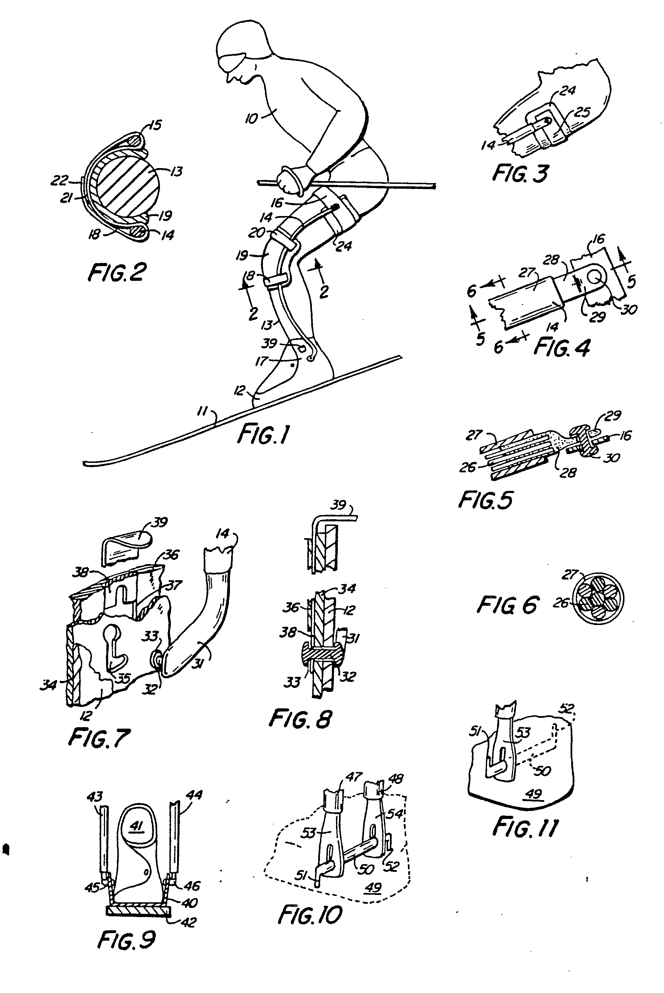

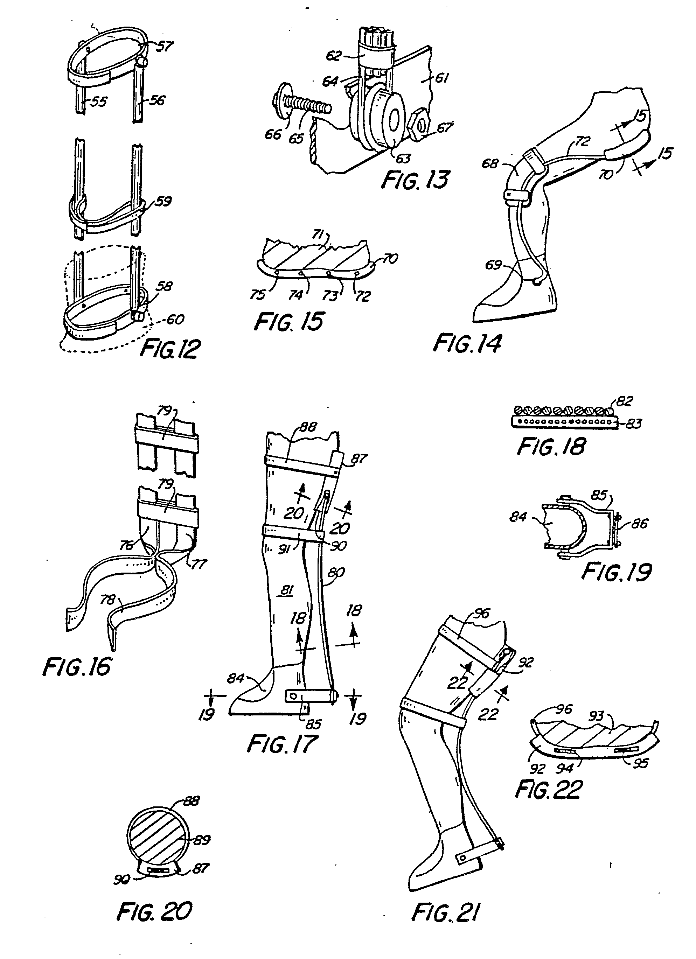

Figures 1 through 15 deal with preferred embodiments which employ laterally disposed biasing members: Figure 1 is a side plan view of a skier equipped with the device; Figure 2 is a sectional view taken on line 2—2 of Figure 1; Figure 3 is a side plan view of a "saddle" upper holder for the thigh area; Figure 4 is an enlarged side plan view of a fastening structure between a biasing member and the upper holder; Figure 5 is a sectional view taken on line 5—5 of Figure 4; Figure 6 is a sectional view taken on line 6—6 of Figure 4; Figure 7 is a fragmentary exploded perspective view of the lower holder at the footwear portion of the device in Figure 1; Figure 8 is a vertical sectional view through the lower holder fastening elements shown in Figure 7; Figure 9 is a front view, partially in section, illustrating a stirrup or under foot arrangement for a lower holder; Figures 10 and 11 are perspective fragmentary views of the heel area of a boot, shown as a phantom fragment, and illustrate two different settings for an alternative under foot lower holder; Figure 12 is a perspective view of a simplified device, with a boot shown as a phantom fragment; Figure 13 is an exploded perspective view of an alternative fastening means between biasing means and a holding means at either the upper or lower end of the biasing means; and Figures 14 and 15 deal with a further variation, with Figure 14 a side plan view and Figure 15 a sectional view taken in line 15—15 of Figure 14.

Figures 16 through 22 deal with embodiments employing biasing means mounted behind a user's leg: Figure 16 is a perspective view, with the upper parts broken away but analogous to those illustrated in Figure 17; Figure 17 is a side plan view of a strip biasing means; Figure 18 is a sectional view, taken on line 18—18 of Figure 17, illustrating two different types of strip biasing members; Figure 19 is a partial sectional view, taken at line 19—19 of Figure 17, and illustrates a lower holder; Figure 20 is a sectional view, taken at line 20—20 of Figure 17, and illustrates the upper holder and slot arrangement for slidable mounting of the upper end of the strip biasing means; Figure 21 is a side plan view with the user's knee partially bent; and Figure 22 is a sectional view, taken on line 22—22 of Figure 21, and illustrates a possible seat arrangement for an upper holder which has two slot recesses for slidably receiving strip biasing means for each leg.

Description of the Preferred Embodiments

In Figure 1, a skier 10 equipped with skis 11 and ski boots 12 is shown with his legs 13 in flexed condition. Therefore the biasing means 14, 15 on his legs is in a yieldingly bent condition. Only one side of one leg is viewable in Figure 1; but the inside lateral part of that leg is equipped with the same elements (see Figure 2) as shown on the outside viewable in Figure 1.

Ordinarily both legs of a user will be equipped with biasing means according to the invention; but for descriptive purposes, the device of the invention will be detailed by making reference to a device for one leg only.

The biasing means comprises two biasing members 14, 15, each of which is elongated and normally substantially straight and always flexibly resilient. The resilience of the biasing members is that of returning from a bent condition to a relatively straight non-stressed condition. They are not mere stretchy elastic members. The two biasing members 14, 15 are along opposite lateral portions of a user's leg. They are in approximate alignment with the user's leg from a level near the ankle area up to at least the level of the mid-thigh area.

The holding means includes an upper holder 16, a lower holder 17, and an intermediate holder comprising straps 18, 20.

The intermediate holder (Figures 1 and 2) suitably has a knee pad 19 which extends on lateral sides of the knee area. Two strap members 18, 20 serve as connectors between the biasing members and function to maintain the mid portion of the biasing members 14, 15 at a location laterally adjacent the knee area. Illustratively, lower strap 18 extends over the lower edge of the knee pad 19, and therefore over the front of the leg, and then about the laterally disposed biasing members 14, 15. Its ends 21, 22 are united together by any suitable fastening means, such as a buckle or a tie or otherwise (e.g., a surface array of knobbed or hooked elements interlockable with a surface array of cooperatively mating character). The intermediate holder maintains the mid portion of the biasing members 14, 15 along opposite lateral portions of the knee area during bending of the knee.

The upper holder 16 maintains upper portions of the biasing members 14, 15 along opposite lateral portions of a user's thigh area. In Figure 1, it is shown as a strap 16 wrapped around the mid-thigh portion of the leg, with ends of the strap fastened together. A pad 24 contributes to comfort.

As illustrated in Figure 3, the upper holder may simply consist of a linking member or strap or contoured plate 25, suitably with pad 24, between the upper ends of the biasing members. It suitably embraces or extends only about the back or rear part of the user's thigh. Since the biasing members 14, 15 are more or less fixed at the footwear or ankle level or area, all that is needed to hold them aligned upon the leg of a user is a means at the knee area for a forward pulling action, and a means at the uppermost ends for a rearward pulling action. The biasing members themselves oppose the pulling forces of these holders, and tend always to urge toward a straightened leg condition.

The biasing members 14, 15 may comprise a plurality of elongated substantially straight flexibly resilient biasing elements or wires 26, closely bunched together, and therefore considered to be a bundle, as particularly illustrated in Figures 5 and 6. Elements 26 may consist of black oil tempered spring wire having a diameter of about 2.66 millimeters. A group of, for example, 3 to 20 such elements (varying depending on the diameter and biasing strength) may be surrounded by a flexible sheath outer covering 27, such as a tube of polyethylene or other flexible plastic or rubbery material. Optionally, they need not be within a sheath; or they may be wrapped in their entirety at spaced locations with any suitable flexible tape. Biasing elements of this type are usually not embedded in any matrix of material for at least the major portion if not all (except their ends) of their length. They optionally may be embedded in a flexible plastic such as, for example, polyethylene or a flexible epoxy resin; but such introduces a dampening effect upon their resiliency, and is in most cases not desired. A non-embedded arrangement allows each wire to shift slightly with respect to its neighbors in the bundle, thereby exhibiting its highest resiliency even at higher bending, without significant dampening and with less tendency toward permanent set at higher bending.

As illustrated in Figure 5, the ends of the elements 26 may be embedded and fixed within a solid mass 28 of metal or of tough sturdy organic plastics material such as, for example, one of phenolic or epoxy or nylon. A ferrule band or cap of metal may be employed at the ends, with or without embedding material. The material at the ends of the biasing elements suitably may be shaped so as to form an ear 29 extending longitudinally outward. A rivet 30 or other suitable fastener may be placed through a hole in the ear 29 and a hole in the upper strap holder 16 (or linking member 25). This allows some slight pivotal movement of the upper ends of the biasing members with respect to the upper holder. The slight pivot action occurs as the user flexes his legs from an extended condition.

The lower holder of Figure 1 comprises fastening means at opposite lateral portions of the footwear of the user. One such fastening means, and the cooperative elements at the lower end of a biasing member for removably fastening the same, is illustrated in Figures 7 and 8. The lower end of the biasing member 14 terminates in a forwardly extending curvature or hook 31 suitably made of metal or any tough sturdy material, including plastics. (The biasing elements within the biasing member 14 are suitably unified at this lower end in a manner comparable to that discussed with respect to Figures 5 and 6.) Hook 31 is provided with an inwardly (toward user) projecting shaft 32 terminating in an expanded annular bulge or flange 33. Embedded in each lateral side of the boot 12 is a plate 34 having an "L" shaped opening 35 in it, with the upper portion of the "L" shape enlarged. The bulge terminus 33 is slipped into the "L" shape and slid along its legs to the lower end of the "L". Behind the plate 34 is a fixed rigid metal housing 36 having a groove 37. Key 38 is hand slidable in groove 37, and is slid down the groove 37 and locked over the bulged knob 33. This fastening means also permits a slight pivot movement between the fastening structure and the end of the biasing member 14.

The biasing members 14, 15 when separated from the skier are substantially straight; but mounting upon the leg of the skier will cause some slight curvature of them even when the skier's leg is in a fully extended condition. In effect, substantially straight biasing members are approximately aligned with the user's leg; and they bias the skier's legs toward a straightened condition without torsion or twisting of the legs. But the holding means (i.e., the upper 16 and lower 17 and intermediate 18 holders) maintain the biasing members approximately aligned upon the user's leg regardless of whether the leg is in flexed or extended condition.

In Figure 9, the lower holder comprises a stirrup 40 attached to the sole of a boot 41 on a ski 42. The lower ends of the biasing members 43, 44 are fastened to the stirrup member by cooperative means such as a nut and bolt assembly 45, 46.

In Figures 10 and 11, the lower holder fastening means consists of a shaft 50 extending transversely through the heel structure and key elements 51, 52 projecting radially out from each end of the shaft. At the lower end of each of the biasing members 47, 48 is an ear 53, 54 having a circular hole and a key slot extending upwardly. The lower ends 53, 54 of the biasing elements are slid upon the shaft 50 with the key elements 51, 52 positioned upwardly as in Figure 11. Then the key elements are turned downward to a locking position as illustrated in Figure 10.

Figure 12 shows two biasing members 55, 56 equipped with the most simple strap-type upper 57, lower 58, and intermediate 59 holders. The lower strap holder is adapted to be wrapped around the ankle area of the boot 60 of a user. The intermediate strap 59 is placed over the forepart of the leg suitably at a location either just above or just below the knee. To keep strap 59 from shifting along biasing members 55 and 56, it may be fixedly fastened as by adhesive to them. In Figure 13, a bundle 62 of resilient biasing elements is shown with at least one resilient element or wire 64 wrapped around a sleeve 63 in a spiral manner and then brought back into the bundle 62. The bundle is suitably taped or equipped with a ferrule ring. The fastening means for fixing the end of the resilient biasing member 62 to the strap holder 61 consists of a bolt 65, washer 66, and nut 67.

Figures 14 and 15 show a device (including holders 68 and 69) identical to that of Figure 1 except for the upper holder. The upper holder comprises a seat member 70 contoured to the buttocks 71 of a user, with the upper ends of the biasing members 72, 73 and 74, 75 suitably fixed to the seat member.

Figure 16 illustrates a biasing support formed out of two solid bands or strips of spring steel or equivalent. Each band 76, 77 is contoured so as to form a clip member 78 to grip the ankle of a user from the rear. The bands of spring metal extend up the rear of the user's leg; and in all other respects, the device in Figure 16 is comparable to that in Figure 17. The two adjacent bands of spring steel may be banded 79 together at intervals, as illustrated.

In Figures 17 through 20, the biasing means comprises a strip biasing member 80 for placement behind the leg 81 of a user. The member 80 may be a single solid band of resilient material. Preferably, it is comprised of a plurality of elongated flexible elements substantially aligned. A flat array strip may solely consist of a single layer 82 (see Figure 18) of elongated flexibly resilient substantially straight biasing elements. Also in Figure 18 is a second flat array 83 of elongated flexible elements (e.g. glass fibers, spring metal wires) bonded and embedded within a flexible plastic, such as, for example, a flexible epoxy. Either such flat array or layer of elements may be employed singly as a strip biasing member; but two or three strip layers as a combination are also useful. The lower holder 84 comprises footwear equipped with a "U" shaped bracket 85. Any suitable clamp 86 or other means may be employed to secure the lower end of the strip biasing member 80 to the bracket 85. (This general arrangement is also useful as the lower holder for the laterally spaced biasing members illustrated in Figure 1.) The upper holder comprises a receiving means 87 equipped with a belt or strap 88 for fastening it to the rear of the upper thigh 89. A slot recess 90 extends into the receiving means 87 and is adapted to receive the upper end of the strip biasing member 80 and allow slide movement (i.e., a longitudinal shift) of that strip biasing member therewithin. The intermediate holder may consist simply of a strap or belt 91 encompassing both the strip biasing member 80 and the leg 81 at a location proximate to the knee and preferably just above the knee.

To be recognized is that as a skier bends a leg equipped with the device of Figure 17, with the holder 91 drawn and fastened, the strip biasing member 80 bends and also slides upwardly in the slot 90 of the receiving member 87. Extension of the leg in turn causes the upper end of the strip biasing member 80 to slide downwardly in the receiving member.

The device illustrated in Figure 21 is identical to that of Figure 17, except for the upper holder illustrated in Figures 21 and 22. Receiving member 92 is especially contoured so as to fit the buttocks 93 of a user. Two slot recesses 94, 95 extend within the receiving member 92, one for the strip biasing member of each leg. A belt or strap 96 fastened to the receiving member 92 is wrapped about the user at his hip area.

To be recognized is that biasing members mounted on opposite lateral sides of a leg are balanced or substantially equal in their biasing tendency, thereby operating to maintain the leg substantially free from torque or twisting or from being pulled to one side or the other during leg flexure. The rear or back of leg position for biasing means also is effective for such result. In most cases, except where one leg is notably weaker than the other, it is preferable that each leg be biased to the same extent.

Different people using a device according to this invention may desire different degrees of weight relief and leg assistance, but operative and effective assistance to improve leg stamina is considered, as a result of testing, to be not less than a minimum total weight "lift" of approximately 3 kilograms for a user whose legs are in a flexed condition of about 90° (i.e., the thigh and lower leg at about a 90° angle).

Stated another way, a minimum total "lift" of about 5% of a user's total body weight should be exhibited by effective biasing means on legs at 90°. As "lift" is increased beyond 70% or so of a user's total weight, even up to the highest "lift" of full body weight, there is introduced possible hazards or possible dangers arising from removing too much control from the leg muscles. Rarely will a "lift" in excess of 70 kilograms be desirable; and such high levels of total "lift" are useful mainly for those of relatively higher body weight. For many applications, a total "lift" in excess of about 5 kilograms, but not over about 45 kilograms, will be found most useful and effective to increase leg stamina with an appropriately high safety factor. This preferred range is useful for persons weighing from about 40 kilograms up to about 90 kilograms.

The amount of "lift" exerted by an individual elongated biasing means (of length from ankle level to the mid-thigh level) is easily approximated by placing one end of it on a weight scale and pressing the other end against the scale until the length of the biasing means is yieldingly bent into approximately a semi-circle. The weight reading (less the weight of the biasing means per se) is considered to be an approximation of its "lift", for it approximately represents the force exerted by the bent biasing means against the force of bending it. The force of bending it, can be considered the approximate body weight "lifted" or supported by it for a 90° knee flexure by the user. It is emphasized that this is but an approximation; the exact location of the biasing member ends on a thigh affects the leverage factor and likewise the true body weight supported.

To be recognized also is that the length of biasing means in use affects the amount of "lift" by it. For example, a biasing member consisting of a bundle of 7 spring steel wires about 2.66 mm in diameter tested to give "lift" of about 11 kilograms for a length of about 63 cm; but the "lift" for this biasing member when at about 90 cm was reduced by almost half. Thus the exact placement of holders when mounting biasing members can affect the lifting function thereof.

Further, as the thickness or diameter of individual wire elements is decreased, the "lifting" power per wire element decreases; but the ability of the wire to undergo greater and greater bending without taking on a permanent set increases. Thus, as the diameter of individual wire elements is decreased, a greater and greater number of such elements must be bundled in order to maintain a lift capacity equal to that for a biasing member of larger diameter units bundled together. This is because, as the cross sectional area of a wire is decreased, its flex strength or resistance to bending decreases much faster than linearly. For example, a ten percent decrease in the diameter of a wire can cause a decrease in flex strength or "lift" of about 30 percent or slightly more. Stated another way, for a decrease of about ten percent diameter, approximately 30 percent more wires of otherwise equal character are required to achieve approximately equal "lift". Even so, it is usually more preferable to employ a bundle of resilient elements than to employ a single solid spring steel rod as a sole biasing member, both from the standpoint of avoidance of permanent set as well as the greater bendability with useful support.

A still further variable arises from the composition or alloy employed to form flexibly resilient elements. This is brought out most dramatically by the fact that an individual resilient element consisting of an epoxy resin bonded group of glass fibers exhibits a relatively weaker "lift" or resistance to flexing as compared to an equal diameter spring steel wire, but a greater degree of bend without taking on a permanent set.

Nevertheless, useful elongated substantially straight flexibly resilient biasing members can be formed by using aligned glass fibers as flexible elongated elements per se and embedding them in a flexible matrix of material, preferably a resinous material such as an epoxy resin. The cross sectional size of round members of this type may be over a half centimeter in diameter up to slightly over a full centimeter in diameter, or slightly more. If the cross section employed is square, the thickness in each direction may be a half centimeter or even a full centimeter or slightly more. Despite the larger cross sectional size as compared a biasing member exhibiting equal lift and consisting of a bundle of resilient metal wires, the glass fiber and resin composites are lighter in weight than an equivalent strength composite biasing member formed using metal wires. Additionally, the glass fiber and resin composites provide resulting biasing members which are highly resistant to taking on a permanent set on repeated bending during leg flexure.

The force or weight required to initiate the bending of biasing elements is less than the force or weight required to increase the bending of the elements at any later stage or degree of bending. Thus, the weight "lifted" or supported increases as the bending is increased beyond a 90° knee flexure or semi-circle for the biasing means; but a point is generally reached, on excessive bending, where danger of a permanent bend set arises. This increased resistance to bending as bending increases exhibits itself beneficially in a variety of ways, as for example, when a skier lands after jumping.

In light of all these variables, the terms "operable" or "effective" or "useful" or "significant" total lift or total support of a portion of body weight become appropriate as a practical and accurate manner of expressing the significant function performed by the biasing members to improve leg stamina. Even more appropriately, the portion of body weight supported is significant to reduce leg strain and improve stamina for repeated flexure.

The variables discussed above are equally applicable to strip or band members. In all cases the significant total lift or partial body support is in the range giving stamina improvement, and will generally be in the total lift range aforementioned. Noteworthy however is the fact that the width of strip biasing means may be relatively great without interference with leg function, even as great as 10 cm or sometimes possibly 15 cm wide. Greater widths allow for relative reduction of thickness while retaining comparable lift.

Users may sometimes desire to mount a device of this invention on their legs before putting on their pants or trousers. The holding means for the biasing means or members may comprise reinforced elements of a person's trousers or underwear or boots. That is, the biasing means or members may be built into apparel for the legs, with reinforced elements of the apparel functioning as the holding means for maintaining operable leg alignment. While non-coiled substantially straight biasing members have been found fully effective and reliable, and are greatly preferred, selective coiling, as for example, a single loop coil at the knee area, may give effective biasing strength and may possibly be employed if the coil bulk is not considered objectionable. The possibility also exists for the employment of members with some hinging rendered inoperable in use. Strap holders, especially for the knee or mid portion of biasing members, may exhibit a slight or limited degree of elasticity, if desired.

**Claims**

1. A leg aid device comprising elongated means for placement upon a user's leg and holding means for maintaining said elongated means upon the user's leg, said elongated means consisting essentially of elongated flexibly resilient biasing means (14, 15) having a length sufficient for placement in approximate alignment with the user's leg from a level near the ankle area up to at least the level of the mid-thigh area and having a flexibility sufficient for said length to be repeatedly yieldingly bent without taking on a permanent set, said biasing means when held upon a body-supporting user's leg by said holding means functioning to support a portion of the user's body weight during the times the user's leg is in a flexed condition and functioning to aid the user's leg muscles in causing extension of the user's leg from a flexed condition, said holding means consisting essentially of a lower holder (17) for fastening the lower end of said biasing means at a level near the user's ankle area and an upper holder (16) for maintaining the upper end of said biasing means at a level at least as high as the user's mid-thigh area, said device being particularly characterized in that said biasing means (14, 15) is capable of having its length repeatedly yieldingly bent into at least a semi-circle without taking on a permanent set while at the same time being sufficiently resistant to bending to provide support for an increasing portion of a user's body weight as the user's leg flexure increases, and by the further fact that said holding means consists also of an intermediate holder (18, 19, 20) for applying a forward pulling force transversely upon said biasing means (14, 15) at a mid-portion thereof at the level of the user's knee area, said intermediate holder being separate from said lower (17) and upper (16) holders but functioning in cooperation therewith to cause said biasing means (14, 15) to urge the user's leg toward a straightened condition without significant torsion or twisting of the user's leg and also functioning in cooperation therewith during leg flexure to cause said biasing means (14, 15) to take on an arcuate contour throughout substantially the entire length of said biasing means.

2. The device of claim 1 wherein said lower holder comprises means (32, 33, 35, 38) for allowing pivot movement of the lower end of said biasing means.

3. The device of claims 1 or 2 wherein said lower holder comprises holding means (35, 38) mounted on or part of the footwear of the user and wherein cooperative means (32, 33) at the lower end of said biasing means is provided for removably fastening said biasing means to said holding means.

4. The device of claims 1, 2 or 3 wherein said upper holder comprises means adapted to embrace the back part of the user's thigh or buttocks (24, 25), and wherein said intermediate holder consists essentially of means (18, 20) for solely contacting the forward portion of the user's leg at or adjacent to the knee area.

5. The device of claims 1, 2, 3, or 4 wherein said biasing means comprises two longated biasing members (14, 15), and wherein said intermediate holder comprises means (18, 20) for maintaining the mid-portion of said biasing members along opposite lateral portions of the user's knee area.

6. The device of claims 1, 2, 3 or 4 wherein said biasing means comprises a strip biasing member (80) for placement behind the leg of a user, and wherein

(i) said lower holder comprises means (85) for maintaining the lower portion of said strip biasing member behind the user's ankle area.

(ii) said upper holder comprises means (87) for maintaining the upper portion of said strip biasing member behind the user's thigh area, and

(iii) said intermediate holder comprises means (91) for drawing a mid-portion of said strip biasing member toward and maintaining the same behind the user's knee area.

7. The device of claims 5 or 6 wherein the structure of each said biasing member(s) comprises a plurality of elongated flexible elements (26, 82) grouped together.

8. The device of claim 7 wherein said elements are individually bendably resilient and not embedded in any matrix for at least the major portion of their length, and wherein at least the ends of said biasing member(s) includes means (28) uniting said elements together.

9. The device of claim 7 wherein said elements are embedded in a flexible matrix (83) of material.

**Revendications**

1. Dispositif d'appui de jambe comprenant des moyens allongés pour le placement de celui-ci sur une jambe de l'utilisateur et des moyens de fixation pour maintenir lesdits moyens allongés sur la jambe de l'utilisateur, lesdits moyens allongés consistant essentiellement en moyens d'inclinaison (14, 15) élastiques, flexibles et allongés ayant une longueur suffisante pour être placés approximativement dans l'alignement de la jambe de l'utilisateur depuis un niveau proche de la zone de la cheville jusqu'à au moins le niveau de la zone de mi-cuisse et ayant une flexibilité suffisante pour que ladite longueur puisse être courbée d'une manière flexible et à de nombreuses reprises sans prendre une forme permanente, lesdits moyens d'inclinaison quand ils sont montés sur la jambe de l'utilisateur comme support de son corps par lesdits moyens de fixation, fonctionnant pour supporter une partie du poids du corps de l'utilisateur durant le temps où la jambe de l'utilisateur est en position fléchie et fonctionnant pour servir d'appui aux muscles de la jambe de l'utilisateur en déterminant l'extension de la jambe de l'utilisateur à partir d'une position flechie, lesdits moyens de fixation consistant essentiellement en un support inférieur (17) pour attacher l'extrémité inférieure. desdits moyens d'inclinaison à un niveau proche de la zone de la cheville de l'utilisateur et un support supérieur (16) pour maintenir l'extrémité supérieure desdits moyens d'inclinaison à un niveau au moins aussi élevé que la zone de mi-cuisse de l'utilisateur, ledit dispositif étant particulièrement caractérisé en ce que lesdits moyens d'inclinaison (14, 15) sont capables d'avoir leur longueur courbée d'une manière flexible et à de nombreuses reprises dans au moins un demi-cercle sans prendre une forme permanente tandis qu'en même temps, ils sont suffisamment résistants au ploiement pour fournir un support pour une partie plus grande du poids du corps de l'utilisateur lorsque la flexion de la jambe de l'utilisateur augmente, et en ce que lesdits moyens de fixation consistent aussi en un support intermédiaire (18, 19, 20) pour appliquer une force de tirage en avant et transversalement sur lesdits moyens d'inclinaison (14, 15) au milieu de celle-ci au niveau de la zone du genou de l'utilisateur, ladite fixation intermédiaire étant séparée desdites fixations inférieure (17) et supérieure (16) mais fonctionnant en coopération avec elles de manière que lesdits moyens d'inclinaison (14, 15) pressent la jambe de l'utilisateur vers une position redressée sans torsion ou retournement significatifs de la jambe de l'utilisateur et fonctionnant aussi en coopération avec eux durant la flexion de la jambe de manière que lesdits moyens d'inclinaison (14, 15) prennent un contour arqué substantiellement dans toute la longueur desdits moyens d'inclinaison.

2. Dispositif selon la revendication 1 dans lequel ladite fixation inférieure comprend des moyens (32, 33, 35, 38) pour permettre le mouvement de pivotement de la partie inférieure desdits moyens d'inclinaison.

3. Dispositif selon l'une quelconque des revendications 1 ou 2 dans lequel ladite fixation inférieure comprend des moyens de maintien (35, 38) montés sur la chaussure de l'utilisateur ou une partie de celle-ci et dans lequel des moyens de coopération (32, 33) à l'extrémité inférieure desdits moyens d'inclinaison sont prévus pour attacher d'une manière amovible lesdits moyens d'inclinaison auxdits moyens de fixation.

4. Dispositif selon l'une quelconque des revendications 1, 2 ou 3 dans lequel ladite fixation supérieure comprend des moyens adaptés pour contenir la partie arrière de la cuisse ou des fesses (24, 25) de l'utilisateur et dans lequel ladite fixation intermédiaire consiste essentiellement en des moyens (18, 20) pour être en contact seulement avec la partie avant de la jambe de l'utilisateur dans la zone du genou ou dans une zone adjacente.

5. Dispositif selon l'une quelconque des revendications 1, 2, 3 ou 4 dans lequel lesdits moyens d'inclinaison comprennent deux éléments allongés d'inclinaison (14, 15) et dans lequel ladite fixation intermédiaire comprend des moyens (18, 20) pour maintenir la partie médiane desdits éléments d'inclinaison le long des portions latérales et opposées de la zone du genou de l'utilisateur.

6. Dispositif selon l'une quelconque des revendications 1, 2, 3 et 4 dans lequel lesdits moyens d'inclinaison comprennent un élément d'inclinaison (80) en ruban à placer derrière la jambe de l'utilisateur et dans lequel

a) ladite fixation inférieure comprend des moyens (85) pour maintenir la partie inférieure dudit élément d'inclinaison en bande derrière la zone de la cheville de l'utilisateur

b) ladite fixation supérieure comprend des moyens (87) pour maintenir la partie supérieure dudit élément d'inclinaison en bande derrière la zone de la cuisse de l'utilisateur et

c) ladite fixation intermédiaire comprend des moyens (91) pour tirer le milieu dudit élément d'inclinaison en bande vers la zone du genou de l'utilisateur et le maintenir derrière cette zone.

7. Dispositif selon l'une quelconque des revendications 5 ou 6 dans lequel la structure de chacun desdits éléments d'inclinaison comprend plusieurs éléments allongés et flexibles (26, 82) groupés ensemble.

8. Dispositif selon la revendication 7 dans lequel lesdits éléments élastiques sont susceptibles d'être courbés séparément et non enfermés dans une gaine sur au moins la majeure partie de leur longueur et dans lequel au moins les extrémités desdits éléments d'inclinaison comprennent des moyens (28) unissant ensemble lesdits éléments.

9. Dispositif selon la revendication 7 dans lequel lesdits éléments sont enfermés dans une gaine flexible (83) de matière.

**Patentansprüche**

1. Beinstütze mit länglichen Teilen zur Plazierung auf dem Bein und mit Halterungen, um die länglichen Teile auf dem Bein zu halten, wobei die länglichen Teile im wesentlichen aus länglichen, nachgiebig elastischen Spannmitteln (14, 15) bestehen, die eine Länge aufweisen, die für eine Plazierung in annähernder Ausrichtung mit dem Bein von der Höhe des Knöchelbereiches bis mindestens zur Höhe des mittleren Schenkelbereiches ausreicht und die eine Elastizität aufweisen, die bei dieser Länge ausreicht, um wiederholt nachgiebig gebogen zu werden, ohne eine dauerhafte Verformung anzunehmen, und wobei die Spannmittel, wenn diese auf dem Bein durch die Halterungen gehalten werden, so wirken, daß sie einen Teil des Körpergewichtes des Benutzers während der Zeit, wenn das Bein in gebeugter Lage ist, stützen und dahingehend wirken, daß sie die Beinmuskeln durch Strecken des Beines aus einer gebeugten Lage unterstützen, wobei die Halterungen im wesentlichen aus einer unteren Halterung (17) zur Befestigung des unteren Endes der Spannmittel in Höhe neben dem Knöchelbereich bestehen und aus einer oberen Halterung (16), um das obere Ende der Spannmittel auf einer Höhe zu halten, die mindestens im mittleren Schenkelbereich liegt, dadurch gekennzeichnet, daß die Spannmittel (14, 15) eine geeignete Länge aufweisen und wiederholt nachgiebig halbkreisförmig verbiegbar sind, ohne eine permanente Verformung anzunehmen, während sie gleichzeitig gegenüber einer Verbiegung ausreichend widerstandsfähig sind, um eine Stütze für den steigenden Anteil des Körpergewichtes des Benutzers zu ergeben, während die Beugung des Beines vergrößert wird und daß die Haltemittel ebenso aus einer mittleren Halterung (18, 19, 20) bestehen zum Anlegen einer nach vorne gerichteten Kraft quer zu den Spannmitteln (15) im Mittelteil derselben in Höhe des Kniebereiches wobei die mittlere Halterung von der unteren (17) und der oberen Halterung (16) getrennt ist, jedoch mit diesen zusammen wirkt, um zu bewirken, daß die Spannmittel (14, 15) das Bein ohne nennenswerte Torsion oder Verdrehung in eine gestreckte Lage zwingen und daß sie ebenso mit diesen während der Beinbeugung zusammenwirkt, so daß die Spannmittel (14, 15) eine gebogene Kontur, im wesentlichen über ihre gesamte Länge, annehmen.

2. Beinstütze nach Anspruch 1, dadurch gekennzeichnet, daß die untere Halterung Mittel (32, 33, 35, 38), um eine Drehbewegung des unteren Endes der Spannteile zu ermöglichen, aufweist.

3. Beinstütze nach Anspruch 1 oder 2, dadurch gekennzeichnet, daß die untere Halterung Haltemittel (35, 38) aufweist, die am oder an einem Teil des Schuhzeuges befestigt sind, wobei zusammen wirkende Mittel (32, 33) an dem unteren Ende der Spannmittel zur lösbaren Befestigung der Spannmittel an den Haltemitteln, vorgesehen sind.

4. Beinstütze nach Anspruch 1, 2 oder 3, dadurch gekennzeichnet, daß die obere Halterung Mittel enthält, die dazu ausgelegt sind, den hinteren Teil des Schenkels oder die Gesäßbacken zu umfassen und wobei die mittlere Halterung im wesentlichen aus Mitteln (18, 20), die nur den vorderen Teil des Beines im oder in der Nähe des Kniebereiches berühren, besteht.

5. Beinstütze nach Anspruch 1, 2, 3 oder 4, dadurch gekennzeichnet, daß die Spannmittel zwei längliche Spannteile (14, 15) aufweisen und daß die mittlere Halterung Mittel (18, 20) aufweist, um den Mittelteil der Spannteile entlang gegenüberliegenden seitlichen Abschnitten im Kniebereich zu halten.

6. Beinstütze nach Anspruch 1, 2, 3 oder 4, dadurch gekennzeichnet, daß die Spannmittel eine Spannschiene (80) zur Plazierung hinter dem Bein des Benutzers aufweisen und daß

(i) die untere Halterung Mittel (85) zum Halten des unteren Teiles der Spannschiene hinter dem Knöchelbereich aufweist,

(ii) die obere Halterung Mittel (87) zum Halten des oberen Teiles der Spannschiene hinter dem Schenkelbereich aufweist und

(iii) die mittlere Halterung Mittel (91) aufweist, um den Mittelteil der Spannschiene zum Kniebereich zu ziehen und um selbige hinter dem Kniebereich zu halten.

7. Beinstütze nach Anspruch 5 oder 6, dadurch gekennzeichnet, daß jedes Spannteil eine Vielzahl von länglichen elastischen Elementen (26, 82), die zusammengruppiert sind, enthält.

8. Beinstütze nach Anspruch 7, dadurch gekennzeichnet, daß die Elemente einzeln biegbar elastisch sind und daß der größte Teil von ihrer Länge nicht in eine Matrix eingebettet ist und daß mindestens die Enden des Spannteiles (der Spannteile) Mittel (28) aufweisen, die die Elemente miteinander verbinden.

9. Beinstütze nach Anspruch 7, dadurch gekennzeichnet, daß die Elemente in eine Matrix (83) aus elastischem Material eingebettet sind.

9

0 039 578

FIG. 1
FIG. 2
FIG. 3
FIG. 4
FIG. 5
FIG. 6
FIG. 7
FIG. 8
FIG. 9
FIG. 10
FIG. 11

1

FIG.12

FIG. 13

FIG. 15

FIG.14

FIG.16

FIG.17

FIG.18

FIG.19

FIG.20

FIG.21

FIG.22